# EUROPÄISCHE PATENTSCHRIFT

(11) **EP 2 618 660 B1**
(45) Veröffentlichungstag und Bekanntmachung des Hinweises auf die Patenterteilung: **26.11.2014**
(21) Anmeldenummer: 11794659.0
(22) Anmeldetag: 20.09.2011
(51) Int. Cl.: A01N 25/22, A01N 35/02, C02F 1/50, C07C 41/58, C07C 43/303

(54) **STABILISIERTE 3,3 DIALKOXY-1-PROPEN - ZUBEREITUNG**
STABILIZED 3,3 DIALKOXY-1-PROPENE PREPARATION
PRÉPARATION STABILISÉE DE 3,3-DIALCOXY-1-PROPÈNE

(30) Priorität: 21.09.2010 DE 102010037691
(43) Veröffentlichungstag der Anmeldung: 31.07.2013
(73) Patentinhaber: Blum, Holger, 9053 Teufen (CH)
(72) Erfinder: Blum, Holger, 9053 Teufen (CH)
(74) Vertreter: Puschmann Borchert Bardehle Patentanwälte Partnerschaft mbB
(86) Internationale Anmeldenummer: PCT/EP2011/066335
(87) Internationale Veröffentlichungsnummer: WO 2012/038433

(56) Entgegenhaltungen:
- WO-A1-96/15668
- WO-A1-96/25040
- DE-A1-102010 037 691

## Beschreibung

Die Erfindung betrifft eine stabilisierte 3,3 Dialkoxy-1-Propen -Zubereitung.

3,3 Dialkoxy-1-Propen wird in vielen verschiedenen Anwendungsfällen verwendet, um Gewässer jeglicher Art von unerwünschten Organismen zu befreien. So lehrt die US-A- 3,690,857, dass die Verkrautung durch Wasserpflanzen in Gewässern und Kanälen bekämpfen kann, ohne die Fischpopulation zu gefährden, in dem man in das Wasser 3,3 Dialkoxy-1-Propen eindosiert. Dieses Verfahren dient zur Kontrolle eine weiten Spektrums von Organismen, die in Wassermedien vorhanden sind und Wasserpflanzen, Wassertiere, Algen, Pilze, Bakterien und dergleichen umfassen. Die Wassermedien können in Kanälen und Seen sowie in Bewässerungssystemen Probleme machen.

Aus der US-A- 5,183,944 ist ferner bekannt, dass wässrige Medien, beispielsweise in Kreislaufsystemen, mit Acrolein behandelt werden können, indem Acroleinacetal mit einer Säure, zum Beispiel Schwefelsäure, zusammengebracht wird und das dabei entstehende gasförmige Acrolein durch ein inertes Gas aus der Deacetalisierunglösung abgeführt und in das zu behandelnde wässrige Medium als Gas eingeleitet wird, um die in dem Medium vorhandenen Organismen abzutöten.

In all diesen Anwendungsfällen ist es ein Problem, dass Verbindung 3,3 Dialkoxy-1-Propen als Ausgangsmaterial gelagert werden muss und dabei mit Feuchtigkeit in Berührung kommen kann.

Verbindungen von 3,3 Dialkoxy-1-Propen sind ungiftige, brennbare klare Flüssigkeiten mit charakteristischen, nicht unangenehmen Geruch welche zum Teil auch als Lebensmittelszusatzstoff Bedeutung erlangt haben. Wird 3,3 Dialkoxy-1-Propen jedoch mit feuchtigkeitskontaminierten Oberflächen in Kontakt gebracht, so zersetzen es sich unter Bildung des giftigen Stoffes Acrolein, welcher die CAS 107-02-8 hat. Überdies weist Acrolein sehr stark Tränen reizende Eigenschaften auf. Da bekanntlich alle technischen Apparaturen in der Nähe von Gewässern welche zum Umschlag,

Lagerung oder zum Fortdosieren von Flüssigkeiten benutzt werden nach einiger Betriebszeit im benetzten Bereich mehr oder weniger feuchtigkeitskontaminiert sind wird die praktische Anwendung von Verbindungen der Formel I durch die gleichzeitig übermäßig erhöhten Forderungen für den Arbeitsschutz des Betriebspersonals blockiert.

Mit dem Auftreten von freien Acrolein einher geht eine Verfärbungsreaktion verbunden mit einer Abnahme des Wirkstoffgehaltes. Versuche haben gezeigt dass die Geschwindigkeit mit der die Verbindungen der Formel I beim Kontakt mit feuchtigkeitskontaminierten Oberflächen Acrolein abspalten steigt in der Reihenfolge von Süßwasser zu Brackwasser zu Seewasser steigt.

Es ist Aufgabe der Erfindung den Wirkstoff 3,3 Dialkoxy-1-Propen in Form einer Zubereitung bereit zu stellen, welche der Abspaltung von Acrolein bei Lagerung/ Umschlag/ Förderung in feuchtigkeitskontaminierten Apparaturen widersteht

Diese Aufgabe wird durch die flüssige stabilisierte 3,3 Dialkoxy-1-Propen-Zubereitung nach Anspruch 1 gelöst.

Die flüssige stabilisierte 3,3 Dialkoxy-1-Propen-Zubereitung ist derart stabilisiert, dass sie mittels einer feuchtigkeitskontaminierten Apparatur, bestehend aus Lagertanks, Rohrleitungen, Armaturen, und Dosierpumpe, appliziert werden kann, ohne dass Acrolein merklich freigesetzt wird. Da die Zubereitung eine Abspaltung von Acrolein in feuchtigkeitskontaminierten Apparaturen widersteht, wird auch Verfärbungsreaktion verbunden mit einer Abnahme des Wirkstoffgehaltes entgegenbewirkt.

Verbindungen der Formel II sind ungiftige Flüssigkeiten mit schwachen zum Teil angenehmen Geruch, welche sich in jedem Verhältnis mit den Verbindungen der Formel I homogen vermischen lassen.

Von Vorteil ist, dass die Anwendung von Verbindungen der Formel II als Bestandteil der Zubereitung die Fischpopulation in Gewässern und Kanälen nicht gefährdet.

Nach einer vorteilhaften Ausgestaltung der Erfindung ist die stabilisierte 3,3 Dialkoxy-1-Propen-Zubereitung dadurch gekennzeichnet, dass R1 gleich H ist und/oder dass R2 gleich C1 oder C2 ist Durch das niedrige Molekulargewicht dieser Zubereitungen ergibt sich in vorteilhafter Weise eine Reduzierung der zur Stabilisierung erforderlichen Menge an der Verbindung der Formel II.

Nach einer vorteilhaften Ausgestaltung der Erfindung ist die stabilisierte 3,3 Dialkoxy-1-Propen-Zubereitung dadurch gekennzeichnet, dass der molare Anteil der Verbindung der Formel II in der Zubereitung ca. 0,5 bis 5 mol % ausmacht.

Die Erfindung wird nun an Hand der nachstehenden Beispiele erläutert:

### BEISPIEL 1

a) Zur Herstellung einer flüssigen ZUBEREITUNG A wurde als Wirkstoff die Verbindung 3,3 Dimethoxy1-Propen, mit der CAS Nummer 6044-68-4 mit dem erfindungsgemäßen Stabilisator, der Verbindung 1,1,1Trimethoxy-Methan mit der CAS Nummer 149-73-5 gemischt, so dass die fertige Mischung 98 Mol% Wirkstoff und 2 Mol% Stabilisator enthielt. Dementsprechend enthalten 100 Gramm der Zubereitung A 2,1 Gramm Stabilisator.
b) Die so hergestellte ZUBEREITUNG wurde anschließend getestet um festzustellen ob sich beim Lagern in einer feuchtigkeitskontaminierten Apparatur freies Acrolein bildet

Die Testapparatur besteht aus einem 1 Zoll Rohrdoppelnippel DIN 2982, Werkstoff Edelstahl AISI 316, mit einem Innendurchmesser von 25,7 mm und einer Rohrlänge von 220 mm. Das Rohr wird beidseitig durch je eine 1 Zoll 6-Kant Kappe ähnlich DIN 2991 unter Verwendung von Teflon-Dichtband verschlossen nachdem 0,15 ml filtriertes Seewasser vom Südhafen der deutschen Nordseeinsel Helgoland eingefüllt worden war. Die so präparierte Testapparatur wurde in horizontaler Lage 3 Wochen bei 18 Grad Celsius gelagert, damit die Feuchtigkeit sich vollständig auf der inneren Edelstahloberfläche des Testrohres verteilen konnte.

Anschließend an diese Vorbereitungsphase wurde eine der beiden 6-Kant Kappen geöffnet und 100 ml der unter a) beschrieben ZUBEREITUNG in das Rohr eingefüllt und dann die 6-Kant Rohrkappe, unter Verwendung von Teflon-Dichtband, wieder aufgesetzt Die so verschlossene, mit ZUBEREITUNG gefüllte, feuchte Testapparatur wurde weitere 3 Wochen bei 18 Grad Temperatur gelagert.

Anschließend an diese Einwirkungsphase wird die Testapparatur geöffnet und ca. ein 1 ml Teil des flüssigen Rohrinhaltes zur Analyse in eine Petrischale gegossen.

Die Auswertung der Probe erfolgte mit Olfaktometrie mittels folgender Bewertungsskala
Grad 0 (kein wahrnehmbarer Acroleingeruch, Geruch der Probe wie die Originalzubereitung)
Grad 1 (wahrnehmbarer Acroleingeruch, mässiger Tränenreiz)
Grad 2 (starker Acroleingeruch, sehr starker Tränenreiz)

### BEISPIEL 2

Zur Herstellung einer flüssigen ZUBEREITUNG B wurde als Wirkstoff die Verbindung 3,3 Dimethoxy-1Propen, mit der CAS Nummer 6044-68-4 mit dem erfindungsgemäßen Stabilisator, der Verbindung 1,1,1Trimethoxy-Methan mit der CAS Nummer 149-73-5 gemischt so dass die fertige Mischung 95 Mol% Wirkstoff und 5 Mol% Stabilisator enthielt Dementsprechend enthalten 100 Gramm der Zubereitung B 5,2 Gramm Stabilisator.

Diese Zubereitung wurde der gleichen Testprozedur unterzogen wie in Beispiel 1 Teil b) beschrieben.

### BEISPIEL 3

Zur Herstellung einer flüssigen ZUBEREITUNG C wurde als Wirkstoff die Verbindung 3,3 Diethoxy-1Propen, mit der CAS Nummer 3054-95-3 mit dem erfindungsgemäßen Stabilisator, der Verbindung 1,1,1 Triethoxy-Methan mit der CAS Nummer 122-51-0 gemischt, so dass die fertige Mischung 98 Mol% Wirkstoff und 2 Mol% Stabilisator enthielt Dementsprechend enthalten 100 Gramm der Zubereitung C 2,3 Gramm Stabilisator.

Diese Zubereitung wurde der gleichen Testprozedur unterzogen wie in Beispiel 1 Teil b) beschrieben.

### BEISPIEL 4

Zur Herstellung einer flüssigen ZUBEREITUNG D wurde als Wirkstoff die Verbindung 3,3 Diethoxy-1Propen, mit der CAS Nummer 3054-95-3 mit dem erfindungsgemäßen Stabilisator, der Verbindung 1,1,1 Triethoxy-Methan mit der CAS Nummer 122-51-0 gemischt, so dass die fertige Mischung 95 Mol% Wirkstoff und 5 Mol% Stabilisator enthielt. Dementsprechend enthalten 100 Gramm der Zubereitung D 5,7 Gramm Stabilisator.

Diese Zubereitung wurde der gleichen Testprozedur unterzogen wie in Beispiel 1 Teil b) beschrieben.

### BEISPIEL 5

Zur Herstellung einer flüssigen ZUBEREITUNG E wurde als Wirkstoff die Verbindung 3,3 Diethoxy-1Propen, mit der CAS Nummer 3054-95-3 mit dem erfindungsgemäßen Stabilisator, der Verbindung 1,1,1 Triethoxy Ethan mit der CAS Nummer 78-39-7 gemischt, so dass die fertige Mischung 95 Mol% Wirkstoff und 5 Mol% Stabilisator enthielt. Dementsprechend enthalten 100 Gramm der Zubereitung E 6,2 Gramm Stabilisator.

Diese Zubereitung wurde der gleichen Testprozedur unterzogen wie in Beispiel 1 Teil b) beschrieben.

### BEISPIEL 6

Zur Herstellung einer flüssigen ZUBEREITUNG F wurde als Wirkstoff die Verbindung 3,3 Diethoxy-1Propen, mit der CAS Nummer 3054-95-3 mit dem erfindungsgemäßen Stabilisator, der Verbindung 1,1,1,1 Tetraethoxy-Methan mit der CAS Nummer 78-09-1 gemischt, so dass die fertige Mischung 98 Mol% Wirkstoff und 2 Mol% Stabilisator enthielt. Dementsprechend enthalten 100 Gramm der Zubereitung F 2,9 Gramm Stabilisator.

Diese Zubereitung wurde der gleichen Testprozedur unterzogen wie in Beispiel 1 Teil b) beschrieben.

### BEISPIEL 7

Zur Herstellung einer flüssigen ZUBEREITUNG G wurde als Wirkstoff die Verbindung 3,3 Dimethoxy-1Propen, mit der CAS Nummer 6044-68-4 mit dem erfindungsgemäßen Stabilisator, der Verbindung 1,1,1,1 Tetramethoxy-Methan mit der CAS Nummer1850-14-2 gemischt, so dass die fertige Mischung 98 Mol% Wirkstoff und 2 Mol% Stabilisator enthielt. Dementsprechend enthalten 100 Gramm der Zubereitung G 2,1 Gramm Stabilisator.

Diese Zubereitung wurde der gleichen Testprozedur unterzogen wie in Beispiel 1 Teil b) beschrieben.

### BEISPIEL I 8

Zur Herstellung einer flüssigen ZUBEREITUNG H wurde als Wirkstoff die Verbindung 3,3 Diethoxy-1Propen, mit der CAS Nummer 3054-95-3 mit dem erfindungsgemäßen Stabilisator, der Verbindung 1,1,1,1 Tretraethoxy-Silan mit der CAS Nummer 78-10-4 gemischt, so dass die fertige Mischung 98 Mol% Wirkstoff und 2 Mol% Stabilisator enthielt Dementsprechend enthalten 100 Gramm der Zubereitung H 3,2 Gramm Stabilisator.

Diese Zubereitung wurde der gleichen Testprozedur unterzogen wie in Beispiel 1 Teil b) beschrieben.

### VERGLEICHSBEISPIEL 1

Zur Herstellung einer flüssigen ZUBEREITUNG J wurde als Wirkstoff die Verbindung 3,3 Dimethoxy-1Propen, mit der CAS Nummer 6044-68-4 als alleiniger Inhaltstoff verwendet.

Diese Zubereitung wurde der gleichen Testprozedur unterzogen wie in Beispiel 1 Teil b) beschrieben.

### VERGLEICHSBEISPIEL 2

Zur Herstellung einer flüssigen ZUBEREITUNG K wurde als Wirkstoff die Verbindung 3,3 Diethoxy-1Propen, mit der CAS Nummer 3054-95-3 als alleiniger Inhaltstoff verwendet

Diese Zubereitung wurde der gleichen Testprozedur unterzogen wie in Beispiel 1 Teil b) beschrieben.

### VERGLEICHSBEISPIEL 3

Zur Herstellung einer flüssigen ZUBEREITUNG L wurde als Wirkstoff die Verbindung 3,3 Dimethoxy-1Propen, mit der CAS Nummer 6044-68-4 mit der Verbindung 2,2 Dimethoxy-Propan mit der CAS Nummer 77-76-9 gemischt, so dass die fertige Mischung 95 Mol% Wirkstoff und 5 Mol% 2,2-DimethoxyPropan enthielt. Dementsprechend enthalten 100 Gramm der Zubereitung L 5,1 Gramm 2,2-DimethoxyPropan.

Diese Zubereitung wurde der gleichen Testprozedur unterzogen wie in Beispiel 1 Teil b) beschrieben.

### VERGLEICHSBEISPIEL 4

Zur Herstellung einer flüssigen ZUBEREITUNG M wurde als Wirkstoff die Verbindung 3,3 Diethoxy-1Propen, mit der CAS Nummer 3054-95-3, mit der Verbindung 2,2 Diethoxy-Propan mit der CAS Nummer 126-84-1 gemischt, so dass die fertige Mischung 95 Mol% Wirkstoff und 5 Mol% 2,2 Diethoxy-Propan enthielt Dementsprechend enthalten 100 Gramm der Zubereitung M 5,1 Gramm 2,2 Diethoxy-Propan.

Diese Zubereitung wurde der gleichen Testprozedur unterzogen wie in Beispiel 1 Teil b) beschrieben.

De Ergebnisse sind in der nachstehenden Tabelle 1 aufgeführt

**TABELLE 1**

| Beispiel | Zubereitung | Bewertungsgrad | Farbe | Relativer Wirkstoff Gehalt%* |
|---|---|---|---|---|
| 1 | A | 0 | hell | >99 |
| 2 | B | 0 | hell | 98 |
| 3 | C | 0 | hell | N.A |
| 4 | D | 0 | hell | >99 |
| 5 | E | 0 | hell | N.A |
| 6 | F | 0 | hell | N.A |
| 7 | G | 0 | hell | >99 |
| 8 | H | 0 | hell | >99 |

| Vergleichsbeispiel | | | | |
|---|---|---|---|---|
| 1 | J | 2 | verfärbt | 78 |
| 2 | K | 2 | verfärbt | 72 |
| 3 | L | 2 | verfärbt | 68 |
| 4 | M | 1 | verfärbt | 73 |

| | | | | |
|---|---|---|---|---|
| * Gehaltsbestimmung durch Vergleich mit einer frisch hergestellten Zubereitung. | | | | |

Wie aus der Tabelle 1 ersichtlich sind nur die Zubereitungen A bis H welche erfingdungsgemäß unter Verwendung von Verbindungen der Formel II hergestellt wurden ausreichend geschützt gegen die Abspaltung von Acrolein bei Lagerung/ Umschlag/ Förderung in feuchtigkeitskontaminierten Apparaturen.

Überdies weisen die Vergleichsproben J bis K zusätzlich zu dem Auftreten von freien Acrolein auch Verfärbungen auf, was mit Minderung des Wirkstoffgehaltes einhergeht, wie aus der letzen Spalte der Tabelle 1 ersichtlich ist

## Patentansprüche

1. Stabilisierte 3,3 Dialkoxy-1-Propen -Zubereitung enthaltend eine Verbindungen der Formel I 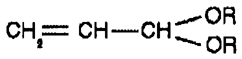 wobei R = C1-C6 Kohlenwasserstoffrest ist, **dadurch gekennzeichnet, dass** die Zubereitung zusätzlich eine Verbindung enthält der Formel II 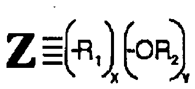 wobei Z = C (Kohlenstoff) oder Si ( Silizium ); R1 = H oder C1-C3 Kohlenstoffrest ; R2 = C1-C3 Kohlenstoffrest; x = 0 oder 1, x+y = 4 ist

2. Stabilisierte 3,3 Dialkoxy-1-Propen-Zubereitung gemäß Anspruch 1, **dadurch gekennzeichnet, dass** R1 gleich H ist

3. Stabilisierte 3,3 Dialkoxy-1-Propen-Zubereitung gemäß Anspruch 1 oder 2, **dadurch gekennzeichnet, dass** R2 gleich C1 oder C2 ist.

4. Zubereitung gemäß einem der Ansprüche 1 bis 3, **dadurch gekennzeichnet, dass** der molare Anteil der Verbindung der Formel II in der Zubereitung ca. 0,5 bis 5 mol % ausmacht.

## Claims

1. Stabilized 3,3 dialkoxy-1-propene -preparation containing a compound of the formula I 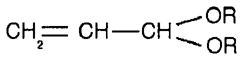 wherein R = C1-C6 hydrocarbon group, **characterized in that** the preparation contains additionally a compound of the formula II 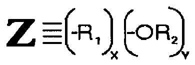 where Z = C (carbon) or Si (silicon); R1 = H or C1-C3 carbon group; R2 = C1-C3 carbon group; x = 0 or 1, x+ y = 4.

2. Stabilized 3,3 dialkoxy-1-propene preparation according to claim 1, **characterized in that** R1 equals H.

3. Stabilized 3,3 dialkoxy-1-propene preparation according to claim 1 or 2, **characterized in that** R2 equals C1 or C2.

4. Preparation according to one of the claims 1 to 3, **characterized in that** the molar portion of the compound of the formula II in the preparation amounts to about 0,5 to 5 mol %.

## Revendications

1. Préparation stabilisée de 3,3-dialcoxy-1-propène comprenant un composé de la Formule 1 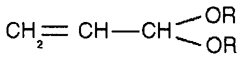 dans laquelle R = C1-C6 est un radical hydrocarboné, **caractérisée en ce que** la préparation contient également un composé de la formule II 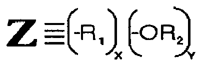 dans laquelle Z = C (carbone) ou Si (silicium) ; R1 = H ou groupe carboné C1-C3 ; R2 = groupe carboné C1-C3 ; x = 0 ou 1, x+y = 4.

2. Préparation stabilisée de 3,3-dialcoxy-1-propène selon la revendication 1, **caractérisée en ce que** R1 est égal à H.

3. Préparation stabilisée de 3,3-dialcoxy-1-propène selon les revendications 1 ou 2, **caractérisée en ce que** R2 est égal à C1 ou C2.

4. Préparation selon l'une des revendications 1 à 3, **caractérisée en ce que** la fraction molaire du composé de la formule II constitue de 0,5 à 5 % molaire de la préparation.
